# EUROPEAN PATENT APPLICATION

(11) **EP 2 081 013 A1**
(43) Date of publication of application: **22.07.2009**
(21) Application number: 09150729.3
(22) Date of filing: 16.01.2009
(51) Int. Cl.: G01N 21/35, G01N 21/88, G01N 33/02

(54) **Method of inspecting food and inspection apparatus implementing the same**

(30) Priority: 18.01.2008 JP 2008009551
(71) Applicant: Sumitomo Electric Industries, Ltd., Osaka-shi, Osaka 541-0041 (JP)
(72) Inventor: Tanaka, Masato, Yokohama-shi Kanagawa (JP); Okuno, Toshiaki, Yokohama-shi Kanagawa (JP); Katayama, Makoto, Yokohama-shi Kanagawa (JP); Shimazu, Takayuki, Yokohama-shi Kanagawa (JP)
(74) Representative: HOFFMANN EITLE

(57) **Abstract**

A food inspection apparatus of the present invention, which allows the improvement of throughput, comprises a light source unit 10 for irradiating near-infrared light to an irradiation range including an inspection object 90; a detection range setting means 60 for setting a detection scope in the irradiation range; a detector unit 20 having a plurality of photodetectors for receiving light such that the light caused in the detection scope by the irradiation is detected repeatedly at intervals of given time; an analyzer unit 30 for extracting a plurality of features by analyzing a signal group which the detector unit 20 outputs according to the detected light intensity; and a display unit 40 for displaying the plurality of features as images.

## Description

### Technical Field

The present invention relates to a method and apparatus for inspecting the quality of a food or the existence/nonexistence of a foreign matter in a food.

### Background Art

In recent years, demand for the safety of food has increased, and therefore, the need for conducting inline analysis about the freshness and quality of the food has been increasing. Inspecting the freshness and quality of food with the naked eye is one way. However, the visual inspection would suffer from differences among individuals, as well as limit in the identification. Moreover, the mixing of foreign matters in foods are serious problems in the production of foods. The mixing of foreign matters occurs in various manners, and when a foreign matter has the same color as the food or is buried in the food, it is difficult to detect with visible light.

The inventions made for the purpose of solving such a problem are disclosed in: for example, Japanese Patent Application Publication Nos. 2004-301690, 2007-010647, 2000-157936, and 2001-099783. At present, a method attracting industrial attention for inspecting the quality of a food and detecting a foreign matter is an analysis using the near infrared light to which food is transparent and which does not suffer from the influence of visible color. Moreover, it is possible to analyze the ingredients of a food by analyzing data obtained wavelengthwise using light of multiple wavelengths.

In the case where a lot of foods are moving on a manufacturing line, for example, it is necessary to process many data at high speed in order to make inline inspection of the foods with near infrared light. Therefore, the improvement in the throughput of food inspection is demanded.

### Disclosure of Invention

### Problem to be solved by the invention

The object of the present invention is to provide a food inspection method and apparatus which allow the improvement of throughput.

### Means for solving the problem to be solved

In order to achieve the object, a method for detecting the existence/nonexistence of a foreign matter in foods or inspecting the quality of foods is provided, wherein the method comprises: a step of irradiating near-infrared light to an irradiation range including an inspection object; a step of setting a detection scope in the irradiation range; a step of receiving light with a detector unit including a plurality of photodetectors such that the light generated within the detection scope by the irradiation is detected repeatedly at intervals of given time; a step of extracting a plurality of features by analyzing a signal group which the detector unit outputs according to the detected light intensity; and a step of displaying the plurality of features as images.

In addition, an apparatus for detecting the existence/nonexistence of a foreign matter in foods or inspecting the quality of foods is provided, wherein the apparatus comprises: a light source unit for irradiating near-infrared light to an irradiation range which includes an inspection object; a detection range setting means for setting a detection scope in the irradiation range; a detector unit which includes a plurality of photodetectors for receiving light such that the light caused by the irradiation to the irradiation range is detected in the detection scope repeatedly at intervals of given time; an analyzer unit for extracting a plurality of features by analyzing a signal group which the detector unit outputs according to the detected light intensity; and a display unit for displaying the plurality of features as images.

### Brief description of the drawings

Figure 1 is a conceptional schematic diagram illustrating an apparatus for inspecting the quality of a food or detecting the existence/nonexistence of a foreign matter in the food.

Figure 2 is a conceptional schematic diagram showing inspection objects, which are being inspected by the inspection apparatus of Fig. 1, and the vicinity thereof.

Figure 3 is a conceptional schematic diagram of another apparatus for inspecting the quality of a food or detecting the existence/nonexistence of a foreign matter in the food.

Figure 4 is a conceptional schematic diagram showing an inspection apparatus according to the embodiment of the present invention.

Figure 5 is a conceptional schematic diagram showing an example of the detection range setting means in the inspection apparatus of Fig. 4.

Figure 6 is a conceptional schematic diagram showing another example of the detection range setting means in the inspection apparatus of Fig. 4.

Figure 7 is a conceptional schematic diagram showing another example of the detection range setting means in the inspection apparatus of Fig. 4.

### Detailed explanation of the invention

Hereinafter, preferred embodiments of the present invention will be described in reference to the accompanying drawings. The drawings are provided for explaining the embodiments and are not intended to limit the scope of the invention. In the drawings, an identical mark represents the same element so that the repetition of explanation may be omitted. The dimensional ratios in the drawings are not always exact.

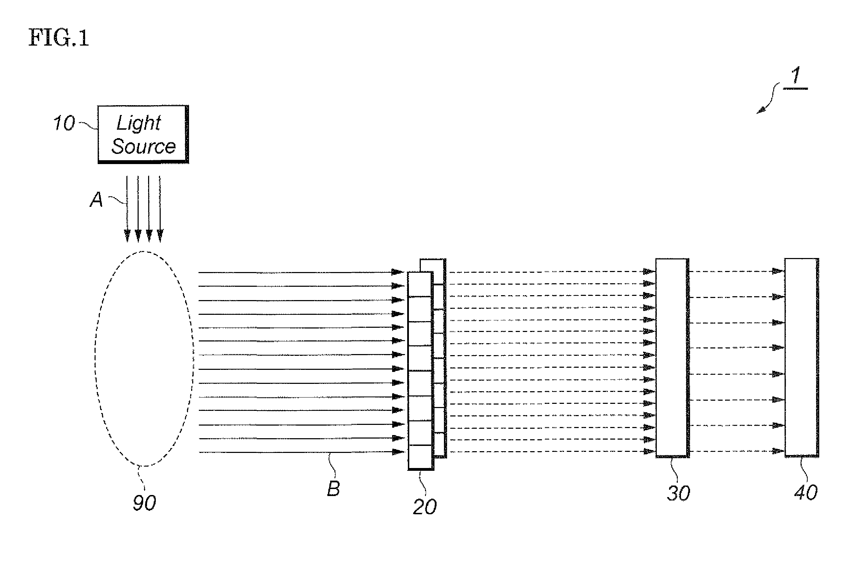

Figure 1 is a conceptional schematic diagram illustrating an apparatus 1 for inspecting the quality of a food or detecting the existence/nonexistence of a foreign matter in the food. The inspection apparatus 1, which is an apparatus for detecting the existence/nonexistence of a foreign matter or inspecting the quality of a food as an inspection object 90, comprises a light source unit 10, a detector unit 20, an analyzer unit 30, and a display unit 40.

The food as the inspection object 90 includes a processed product as well as a material being handled in a manufacturing process. In addition, there may be a case where the inspection object 90 includes a foreign matter or a contaminant besides the food, and the examples of the foreign matter and contaminant includes a hair, a fiber, a remainder of a material (e.g., a skin of a plant, a seed, a bone of an animal, etc.), and an insect. In some cases, the inspection object 90 is contained in a container. It is preferable that the inspection object 90 be moving on a conveyor or in the air during the inspection.

The light source unit 10 irradiates light A in the near-infrared region to an area in which the inspection object 90 lies. The light A includes at least a wavelength component within the wavelength range of 900 nm to 2500 nm, and besides it may also contain a wavelength component outside the wavelength range of 900 nm to 2500 nm.

The detector unit 20 including a plurality of photodetectors receives light by detecting, repeatedly at intervals of a given time, the spatial distribution of light B that is caused in the irradiation range of the light A when the irradiated light A is transmitted through, reflected at, or scattered from the inspection object 90. The detector unit 20 is constituted by arranging a plurality of photodetectors consisting of semiconductors such as InGaAs, Mercury Cadmium Telluride (MCT), PbSe, InSb, etc. which can detect light in the near-infrared region.

The analyzer unit 30 extracts a plurality of features by analyzing signal groups output from the detector unit 20 according to the detected light intensity. For example, the processing modes of output from the analyzer unit 30 are as follows: (Mode 1) the output is made in a color tone converted from the intensity of light detected by each photodetector of the detector unit 20; (Mode 2) the output shows whether the detected light intensity of each photodetector of the detector unit 20 meets a pre-determined condition (e.g., binary value display); and (Mode 3) the detected light intensities of the photodetectors of the detector unit 20 are shown by classifying according to pre-determined conditions (e.g., grouping of the linked pixels). Also, it is desirable that prior to these processing, the analyzer unit 30 be subjected to elimination of noise and the improvement of contrast by image processing such as median filtering and Laplacian filtering to make the most of two-dimensional information.

The display unit 40 displays in an image mode the features obtained as a result of the extraction made by the analyzer unit 30. Here, the display mode of the display unit 40 may be a mode to display a result of each pixel, or may be a mode to display the number of the pixels that have met the specific pre-determined conditions according to the above-mentioned second or third mode of the analyzer unit 30. Also, when the output is made in a numerical form, the analyzer unit 30 may be designed to give an alarm sound or to send a trigger signal to another equipment. For example, if a trigger signal is sent to a remover, the remover will be able to remove the foreign matter detected as a foreign matter.

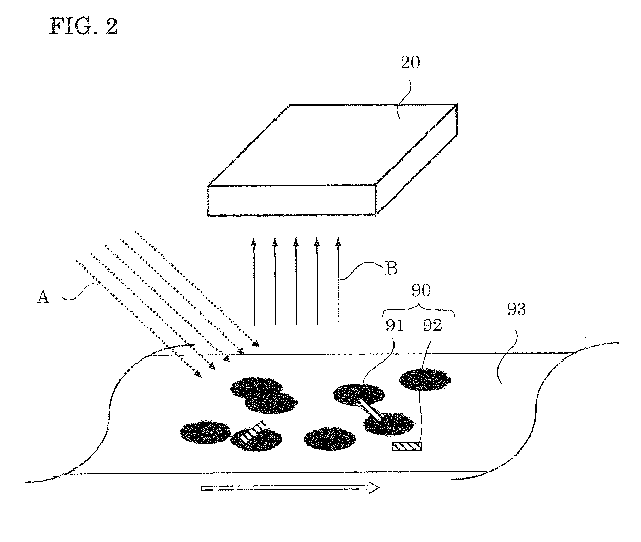

Figure 2 is a conceptional schematic diagram showing inspection objects, which are being inspected by the inspection apparatus 1, and the vicinity thereof. The inspection objects 90 including foods 91 and foreign matters (including contaminants) 92, which are put on a belt conveyor 93, are moving in parallel in a constant direction. Near-infrared light A output from the light source unit 10 is irradiated to the region which includes the inspection objects 90. The light B which arises according to the irradiation in the irradiation range of light A is repeatedly detected at intervals of given time and received by the detector unit 20 having a plurality of photodetectors arranged in an array form.

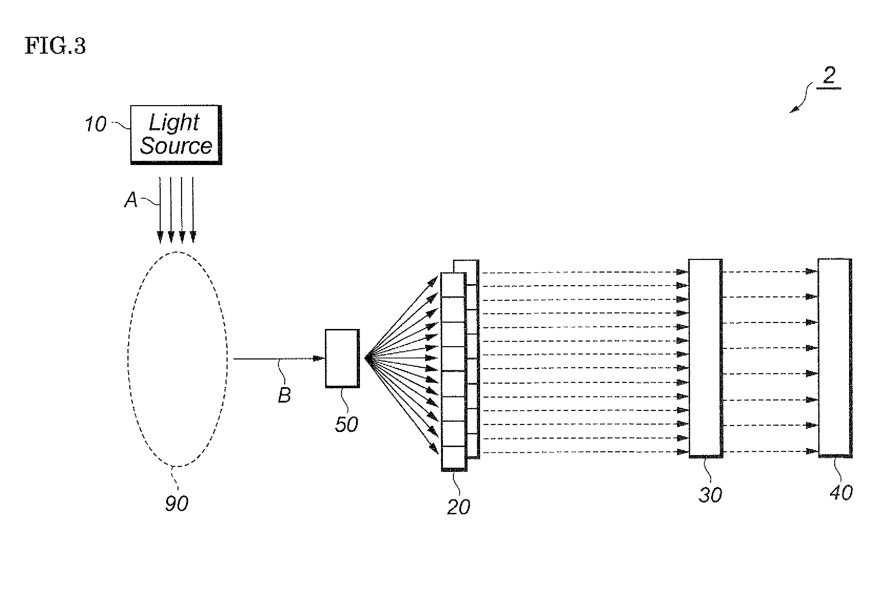

Figure 3 is a conceptional schematic diagram of another apparatus 2 for inspecting the quality of a food or detecting the existence/non-existence of a foreign matter in the food. The inspection apparatus 2, which is an apparatus for inspecting the quality of a food as an inspection object 90 or detecting the existence/nonexistence of a foreign matter in the food, comprises a light source unit 10, a detector unit 20, an analyzer unit 30, a display unit 40, and a spectroscope unit 50.

Light B, which is caused in the irradiation range of the light A by the irradiation of the light A emitted from the light-source unit 10, is separated wavelength-wise by the spectroscope unit 50 which is provided between the inspection object 90 and the detector unit 20. The detector unit 20 receives light having the respective wavelength components separated by the spectroscope unit 50 and outputs signals for showing the spectrum of the light B. The spectrum signals thus output from the detector unit 20 are analyzed by the analyzer unit 30.

In this case, it does not matter whether the light B which is input to the spectroscope 50 has arisen from one region or a plurality of regions of the inspection object 90. In the latter case, with respect to the latter stages after the detector unit 20, the signals may be treated as the two dimensional information consisting of wavelength and position axes.

The output modes of the analyzer unit 30 include a mode in which the strength of a selected wavelength or wavelength band is converted into a color tone for display and a mode in which the result quantified by a calibration curve is shown. It is desirable that prior to these treatments in the analyzer unit 30, the noise be removed and the variation be decreased by performing pre-spectrum processing such as smoothing, baseline correction, or second derivation.

When a large number of inspection objects 90 which move on a line is to be inspected in-line by the inspection apparatus 1 and 2, the analyzer unit 30 must process much data at high speed. Therefore, it is sought to reduce the signal treatment time and to improve the throughput of the food inspection.

When foods as the inspection objects 90 are to be inspected through their images, in some cases an extremely large number of inspection objects 90 having irregular shapes must be inspected while they are placed in a disorderly manner. Also, there may be a case where processed products standing in a row on a manufacturing line must be inspected simultaneously for a plurality of manufacturing lines. Furthermore, in some case, as in the case of the inspection objects 90 having a slender shape, the region that requires a high resolution image with respect to a specific direction only is continuous. In any of those cases, if all regions are inspected in a uniform manner, it will result in redundant inspection data. Also, in the case of spectrum inspection of foods, the optical absorption that occurs due to the existence of ingredients of each inspection object 90 depends on a limited band. Consequently, if a continuous spectrum is detected, it will result in including unnecessary data. Therefore, it would be desirable to make efficient detection by limiting the detecting range by providing a detection range setting means with which the range of the detection to be made by the detector unit 20 can variably be set in the irradiation range of light A.

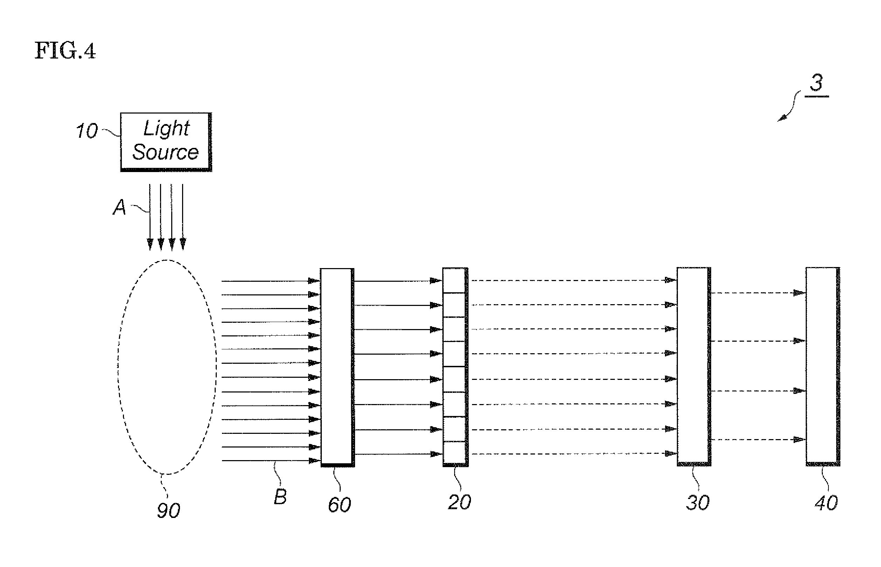

Figure 4 is a conceptional schematic diagram showing an inspection apparatus 3 relating to the embodiment of the present invention. The inspection apparatus 3 is an apparatus for inspecting the existence/nonexistence of a foreign matter in, or the quality of, a food as an inspection object 90, and comprises a light-source unit 10, a detector unit 20, an analyzer unit 30, a display unit 40, and a detection range setting means 60.

The detection range setting means 60, which is disposed between the inspection object 90 and the detector unit 20, can variably set the range for the detector unit 20 to detect the light B in the irradiation range of light A. That is, the detector unit 20 can detect light B selectively in a detection range that is narrowed down from the irradiation range of light A by the operation of the detection range setting means 60. With the structure described above, it is possible to make the detector unit 20 to perform detection while pinpointing only to an inspection object 90 in the whole irradiation range. Therefore, the amount of data to be processed is reduced, which results in improvement in the inspection throughput of foods as the inspection objects 90.

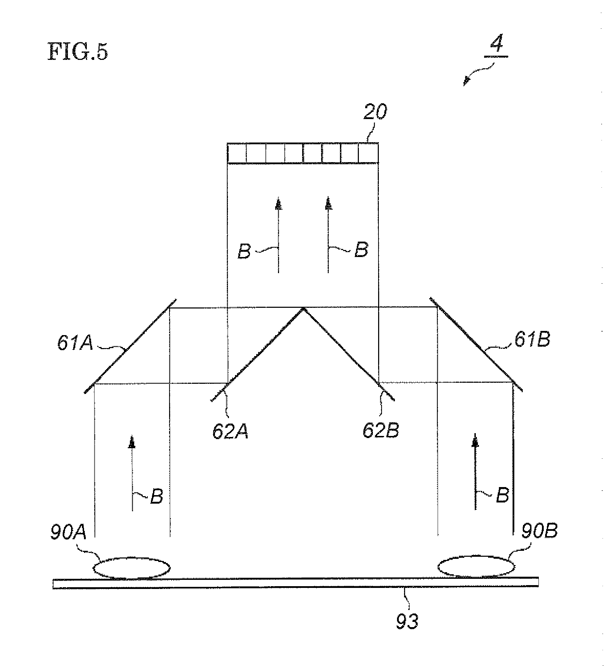

Figure 5 is a conceptional schematic diagram showing an example of the detection range setting means 60. In this example, the detection range setting means 60 is equipped with mirrors 61A, 61B, 62A, and 62B. The mirrors 61A, 61B, 62 A, and 62B function so that the detection scope of the detector unit 20 may consist of two or more partial ranges, and thereby it is made possible for the detector unit 20 to detect the light B having arisen from the inspection objects 90A and 90B which lie mutually apart in such two or more partial ranges.

That is, the light B that has arisen from an inspection object 90A on one side is reflected by the mirror 61A and the mirror 62A in the named order so as to fall incident on a first region of the light-incident face of the detector unit 20. Likewise, the light B that has arisen from another inspection object 90B on another side is reflected by the mirror 61B and the mirror 62B in the named order so as to fall incident on a second region of the light-incident face of the detector unit 20. In this case, the first region and the second region of the light-incident face of the detector unit 20 do not overlap each other. In addition to the mirrors 61A, 61B, 62 A, and 62B, a lens may be arranged on the optical path of the light B between the inspection objects (90A, 90B) and the detector unit 20.

The above-described structure, which does not need a plurality of detector units, will result in cost reduction. Furthermore, since light B having arisen from a plurality of inspection objects is detected by one detector unit 20, the amount of data to be processed will be reduced, which allows improvement in the inspection throughput of foods as the inspection objects. Also, such structure will make it easy to make relative comparison.

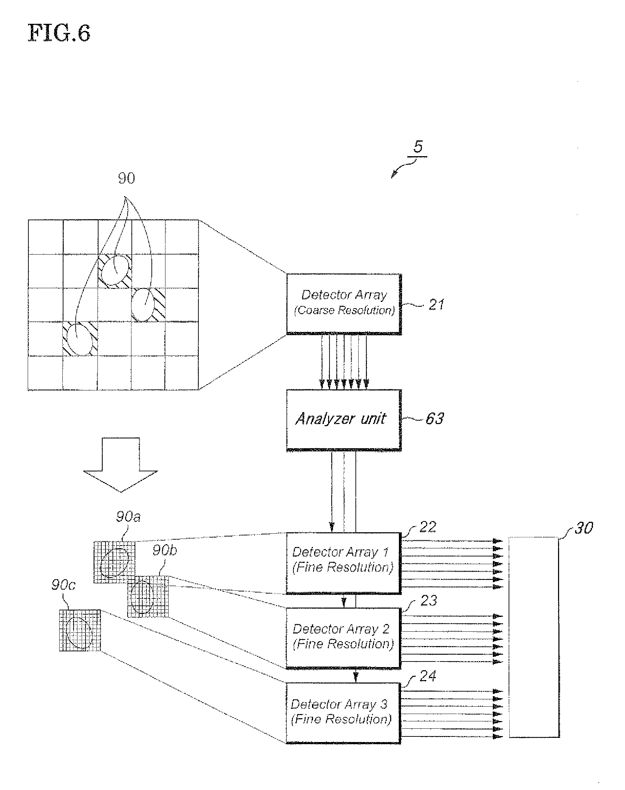

Figure 6 is a conceptional schematic diagram showing another example of the detection range setting means 60. In this example, the detection range setting means 60 has a first detector array 21 and a second analyzer unit 63, while the detector unit 20 consists of second detector arrays 22 to 24. The first detector array 21 receives the light B caused by the irradiation in the irradiation range of light A. The ranges in which the light intensity detected by the first detector array 21 falls within a preset range is determined as partial ranges 90a to 90c in the irradiation range of light A by the second analyzer unit 2. Second detector arrays 22 to 24 selectively receive light B that has arisen in the partial ranges 90a to 90c, respectively, as determined by the second analyzer unit 63.

That is, at a first step, the first detector array 21 detects a wide range including the inspection objects 90. The first detector array 21 used for this purpose may have a coarse resolution, which may be the same size as the inspection object 90. The positions of the inspection objects 90 are determined by such detection. At a second step, the second detector arrays 22 to 24 having high resolution are moved on the basis of the approximate information about the positions of the inspection objects 90 to the positions thus determined. In stead of such arrangement, a mirror may be placed in front of the detector arrays so as to face the objective position. Or, one detector array 22 may be moved so as to continuously detect all objects.

This enables high resolution measurement over a wide scope. The subsequent processing by the analyzer unit 30 is accomplished on the basis of signals output by the second detector arrays 22 to 24. In such manner, the volume of data to be processed can be reduced, and accordingly the improvement in the inspection throughput of foods, which are inspection objects, can be achieved.

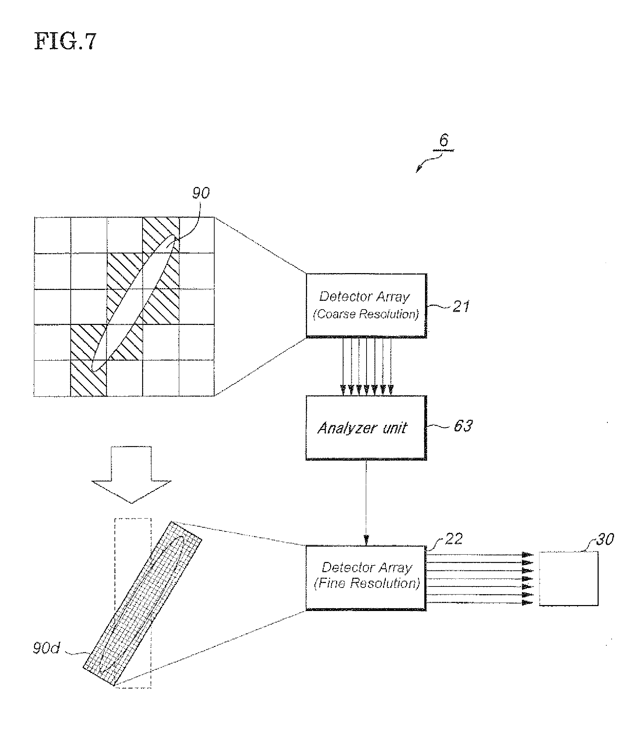

Figure 7 is a conceptional schematic diagram showing another example of the detection range setting means 60. In this example, the detection range setting means 60 is equipped with a first detector array 21 and a second analyzer unit 63, and the detector unit 20 comprises a second detector array 22. The first detector array 21 receives light B caused by the irradiation in the irradiation range of light A. The second analyzer unit 63 determines a partial range, as well as the azimuth thereof, in which the light detected by the first detector array 21 has an intensity that falls within the range previously set in the irradiation range of light A. On the basis of the partial range and the azimuth as determined by a second analyzer unit 63, the second detector array 22 selectively receives light B that has arisen from the partial range.

That is, at a first step, the first detector array 21 detects a wide range including the inspection object 90. The first detector array 21 used for this purpose may have a coarse resolution, which is the same size as the inspection object 90. The position and azimuth of the inspection object 90 are determined by such detection. At a second step, on the basis of the approximate positional information of the inspection object 90, the direction of the high resolution second detector array 22 is adjusted, and the second detector array 22 selectively receives the light B whose aspect ratio is 2 times or more or 1/2 or less and which arises from the partial range 90d in the azimuth determined as described above.

The subsequent processing by the analyzer unit 30 is accomplished on the basis of signals output by the second detector array 22. Thus, with an inspection apparatus 6, it is also possible to reduce the volume of data to be processed and accordingly to achieve the improvement in the inspection throughput of foods as the inspection objects.

The present invention is not limited to the above-described embodiments, and various modifications are possible. For example, in the third embodiment and the embodiments following thereto, a spectroscope unit 50 may be provided between the inspection object 90 and the detector unit 20 as in the case of the second embodiment, and the light B caused by the irradiation of light A in the irradiation range of the light A may be separated with the spectroscope unit 50 so that the detector unit 20 may receive light of each wavelength component upon separation of light.

## Claims

1. An inspection method for detecting the existence/nonexistence of a foreign matter in foods or inspecting the quality of foods, the method comprising:
a step of irradiating near-infrared light to an irradiation range including an inspection object;
a step of setting a detection scope in the irradiation range;
a step of receiving light with a detector unit including a plurality of photodetectors such that the light generated within the detection scope by the irradiation is detected repeatedly at intervals of given time;
a step of extracting a plurality of features by analyzing a signal group output by the detector unit according to the detected light intensity; and
a step of displaying the plurality of features as images.

2. An inspection method according to claim 1, further comprising a step of separating wavelength-wise the light caused by the irradiation in the detection scope,
wherein the detector unit receives a respective wavelength component thus separated at the step of receiving light.

3. An inspection method according to claim 1,
wherein the spatial distribution of the light caused by the irradiation in the detection scope is detected at the step of receiving light.

4. An inspection method according to claim 3,
wherein at the step of setting a detection scope, a partial range having an aspect ratio of 2 times or more or 1/2 or less is set as the detection scope.

5. An inspection method according to claim 1,
wherein two or more partial ranges are set as the detection scope at the step of setting a detection scope, and light caused in the two or more partial ranges by the irradiation are collectively received by the detector unit at the step of receiving light.

6. An inspection method according to claim 1,
wherein at the step of setting a detection scope, the light caused by the irradiation in the irradiation range is received by a first detector array, and a partial range detected in the irradiation range by the first detector array and having a light intensity falling within a preset range is set as the detection scope.

7. An apparatus for detecting the existence/nonexistence of a foreign matter in foods or inspecting the quality of foods, comprising:
a light source unit for irradiating near-infrared light to an irradiation range including an inspection object;
a detection range setting means for setting a detection scope in the irradiation range;
a detector unit including a plurality of photodetectors for receiving light such that the light caused in the detection scope by the irradiation is detected repeatedly at intervals of given time;
an analyzer unit for extracting a plurality of features by analyzing a signal group output by the detector unit according to the detected light intensity; and
a display unit for displaying the plurality of features as images.

8. An inspection apparatus according to claim 7, further comprising a spectroscope unit to wavelength-wise separate light having arisen in the detection scope due to the irradiation,
wherein the detector unit receives light of each wavelength component after such light separation.

9. An inspection apparatus according to claim 7,
wherein the detector unit detects spatial distribution of light caused by the irradiation in the detection scope.

10. An inspection apparatus according to claim 7,
wherein the detection range setting means comprises a first detector array and a second analyzer unit, the first detector array receiving light caused by the irradiation in the irradiation range, the second analyzer unit setting a detection scope in the irradiation range.
